# EUROPEAN PATENT SPECIFICATION

(11) **EP 1 161 670 B1**
(45) Date of publication and mention of the grant of the patent: **10.12.2008**
(21) Application number: 00913516.1
(22) Date of filing: 18.02.2000
(51) Int. Cl.: G01N 15/06, G01N 33/06

(54) **MEASUREMENT SYSTEMS AND METHODS FOR DETERMINING COMPONENT PARTICLE CONCENTRATIONS IN A LIQUID**
MESSVORRICHTUNG UND VERFAHREN ZUR BESTIMMUNG VON TEILCHENKONZENTRATIONEN IN EINER FLÜSSIGKEIT
SYSTEMES ET PROCEDES DE MESURE PERMETTANT DE DETERMINER LES CONCENTRATIONS EN PARTICULES COMPOSANTES D'UN LIQUIDE

(30) Priority: 19.02.1999 US 120858 P; 19.02.1999 US 120857 P
(43) Date of publication of application: 12.12.2001
(73) Proprietor: Metron Instruments, Inc., Solon, OH 44139 (US)
(72) Inventor: LOVETTE, Spencer, M., Katonah, NY 10536 (US); FORD, Norman, C., Jr., Amherst, MA 01002 (US)
(74) Representative: Burrows, Anthony Gregory
(86) International application number: PCT/US2000/004177
(87) International publication number: WO 2000/049388

(56) References cited:
- EP-A- 0 947 822
- US-A- 3 901 602
- US-A- 4 265 538
- US-A- 4 766 083
- US-A- 4 980 295
- US-A- 5 229 839
- US-A- 5 305 073
- US-A- 5 343 044
- US-A- 5 620 000

## Description

This invention relates to measurement systems and methods and, more particularly, to measurement systems and methods for determining casein particle concentration in a dairy product, utilising light scattering techniques. The invention is particularly useful for determining fat and casein concentrations in a dairy product.

It is frequently desirable to determine particle concentration in a liquid. Such measurements may be utilized in process control, research, and the like. In some cases, the liquid may contain particles of one type having one size distribution. In other cases, the liquid may contain particles of two or more types, each having a characteristic size distribution. In the latter case, determining particle concentrations is difficult because of interaction between measurements. An example of a liquid having two types of particles with different size distributions is milk and other dairy products.

Raw milk prices are determined by the fat content of the milk. In most retail markets, 25 dairy processors standardize milk to various levels of fat, e.g., 1%, 2% and 3.25% in the United States. Also in cheese production, quality and yield can be optimized by standardizing the ratio of casein to fat in the milk used to make the cheese. Consequently, there is a need for an effective means of measuring fat and casein concentrations in milk. Standardization requires a robust measurement suitable for operation on the dairy production floor for process control applications.

Several automated techniques have been developed for measuring fat concentration in milk. Currently, measurement of the optical turbidity of milk provides the most accurate and stable instrument-based fat measuring technique. Simple broadband light attenuation measuring detectors are used. The milk sample is homogenized and is diluted with a high pH diluent, such as sodium hydroxide, to dissolve calcium caseinate from the milk, so that it does not interfere with the fat measurement. As far as the Applicant is aware, there are no accepted simple instrumentation methods for measuring casein concentration in milk.

Light scattering is a known technique for characterizing particles in a liquid. In a light scattering system, a liquid containing particles is passed through a sample cell having windows. A light beam is directed through the liquid, and light scattered by the particles in the liquid is analyzed to determine the characteristics of the particles. In one prior art system, the liquid sample is surrounded by an array of detectors which collect laser light scattered by the sample at different angles. In another prior art system, laser light scattered by the sample at a predetermined angle passes through an annular aperture and is focused on a photomultiplier.

Patent document US 5,343,044 A describes a method by quantitatively determining, among others, the concentration of casein in a milk sample by an infrared attenuation measuring system, the system comprising infrared attenuation measuring means for measuring infrared attenuation in a number of "wavebands" (meaning wavenumber ranges). A calibration method is performed by measuring, at different wavebands including, among others and at least for each component to be measured, a waveband at which the component in question influences the infrared attenuation. The degree of homogenisation of the sample is taken into account.

A light scattering, system for molecular characterization is disclosed in US-A-5,305,073 described in more detail hereinafter.

US-A-3,869,209 discloses a system in which the amount of the dispersed phase in a suspension is determined by causing the suspension to flow in a free-falling jet, directing a beam of light transversely on the jet, and measuring the light scattered and otherwise deflected by the jet by means of at least two photoelectric cells placed about the jet outside the incident light beam and its portion linearly transmitted by the jet. It states that three fixedly arranged photoelectric cells permit useful readings on dispersions of a wide variety of materials. In an embodiment, the incident beam and its transmitted portion are at an angle of about 60° to the axis of the jet which is vertical, although that angle is not critical. Solid or liquid particles of a distinct second phase dispersed in the liquid of the jet scatter light in all directions, but the intensity of the scattered light varies with its angular relationship to the optical axis of the incident beam. A first collecting lens is arranged to receive some of the scattered light and to direct it onto a first photoelectric, selenium cell which generates an electrical signal commensurate with the intensity of the scattered light received. The axis of the transmitted portion of the beam and the optical axis of the first collecting lens define an acute angle in a vertical plane containing the axis of the transmitted portion and the axis of the jet. That apparatus is stated to be eminently suitable for measuring the amount of dispersed butter fat in heavy cream or of micro-organisms dispersed in a clear aqueous medium, when the entrance angle of the lens is chosen to cover the range of most intense scattered light. The acute angle between the axis of the transmitted portion and the optical axis of the lens is preferably between 15° and 40° when microbial cells or optically similar materials constitute the disperse phase in the tested suspension.

In one version, the optical axis of a second collecting lens coincides with that of the first collecting lens, the two lenses being arranged on respective opposite sides of the jet, so that the second collecting lens mainly receives light reflected by the jet and focuses the received light onto a second photoelectric cell. Generally, the angle defined by the axis of the second collecting lens and the axis of the transmitted portion of the incident light beam should be at least 90°, but may be as great as 150°, although a maximum of 135° is said to be adequate in many cases. Output circuits electrically connected to the respective photoelectric cells lead to separate indicating devices each equipped with a galvanometer from which the amount of light received by the associated photoelectric cell may be read. If the dispersion tested contains mineral matter dispersed in an aqueous fluid also carrying microbial cells, the concentration of the microbial cells alone can be calculated from the readings of the two galvanometers and a chart based on imperical data, the reading obtained from the first indicating device being corrected for the influence of the suspended mineral matter as read from the second indicating device.

US-A-3,869,209 discloses that, however, it has been found that some dispersions such as skim milk reflect a sizeable portion of incident light in a direction having a major component opposite to the incident beam. It states that, if the apparatus described above is employed for measuring the concentration of skim milk solids dispersed in an aqueous carrier liquid, the amount of light received by the second photoelectric cell is of a similar order of magnitude as the light impinging upon the first photoelectric cell, so that useful measurements cannot be obtained without combining the readings of both indicating devices according to imperical charts which need to be established for each type of tested suspension.

In a second version in US-A-3,869,209, a third collecting lens is provided which receives light from the area of intersection of the incident light beam and the jet and focuses the received light on a third photoelectric cell, the output circuit of which is connected to a third indicating device. It states that useful values of the amount of disperse phase in the jet can be obtained from virtually any type of dispersed material and any type of carrier liquid if the angle between the axis of the transmitted beam portion and the optical axis of the third collecting lens is approximately 90°, a range of 75° to 105° being permissible under most circumstances. It goes on to state that the angular relationship of the third collecting lens to devices other than the incident beam and its transmitted portion is unimportant.

US-A-3,901.602 discloses a light scattering system for chemically identifying individual particles of matter or multiple particles of matter, such as are found in aerosols, without collecting and chemically analyzing the material. In the case of single particle analysis, plane-polarized light is impinged on the particle and the intensity of the light scattered into the plane of polarization over a specified angular range is measured. The intensity is related to the coefficient of absorption and the size of the particle. In multiple particle analysis, the intensity of the light scattered into a plane perpendicular to the plane of polarization is also measured to determine the total number of particles of matter. This information may be used to normalize the intensity measurement of a first scattered light beam. The system may be a smoke detection apparatus embodying the multiple particle analysis technique whereby fire-produced aerosols may be detected without interference from non-fire-produced aerosols of similar density.

US-A-4,265,538 discloses an optical analyzing system in which a rectangular sample cell for use in the measurement of the light scattered from an incident light beam in three mutually orthogonal directions is constructed with a prism having its base optically in contact with the sample along a side of the cell. Two other faces of the prism are oriented at equal angles to the base so that those faces pass orthogonal components of the light scattered at 90° from the axis of the incident beam. The other orthogonal component is formed by the forward scattered light.

US-A-4,907,884 discloses a system for monitoring the intensity of light incident upon a scattering source so that the scattered light intensities recorded therefrom may be normalised accurately to the radiation flux incident thereon. The monitoring of the incident light source is achieved by means of two detectors; one monitors a fraction of the incident light before it enters the cell and the second monitors the light after it traverses and emerges from the cell. The square root of the product of these two values is then used to normalise each detected scattered signal by the light incident on the scattering sample by dividing each such detected scattered signal by that square root value. The square root of the product of these two measurements is claimed to be proportional to the incident intensity at the sample.

US-A-4,980,295 discloses a process for determining quickly and accurately the fat content of food or non-food products which solubilizes the fat content of the product in tetrachloroethylene. The process generally involves extracting the fat from a product to be tested for fat content with tetrachloroethylene, dissolving the fat/tetrachloroethylene solution in a polar organic solvent such as acetic acid, treating the resulting solution with an aqueous surfactant in order to transfer the fat content of the solution to the aqueous surfactant and thereby inducing the formation of fat globules in a resulting suspension and then testing the resulting suspension for monochromatic light dispersion. The light dispersed at the maximum turbidity of the sample is compared to a standard curve plot produced by previous tests on tetrachloroethylene solutions of a pure fat taken from samples comparable to the product being tested. The testing of some products may also require that methanol also be employed in conjunction with the tetrachloroethylene fat solvent. The process may be employed in the meat, dairy, oilseed and animal feed industries.

US-A-5,305,073 discloses a molecular characterization detector which includes a scattering cell containing a sample for molecular characterization, a light source for directing a light beam through the cell so that the light beam is scattered by the sample, optical elements for selecting from the scattered light a measurement beam comprising light that is scattered by the sample from a predetermined portion of the cell in a predetermined range of angles relative to the optical axis, and a detector for detecting the measurement beam and providing an output electrical signal representative of the measurement beam. The detector typically selects light scattered from a central portion of the scattering cell at angles in the range of 14° to 16°. A single spherical lens is preferably utilized. As a result, interference from stray scattered light is minimized. A beam dump attenuates the light beam after it passes through the scattering cell. Additional detectors detect light scattered at 90° to the light beam. The outputs of the detectors are processed to provide molecular characterization of the sample. The detector is usable particularly in control laboratories, research laboratories and manufacturing operations in the plastics, pharmaceutical, biotechnical and chemical industries.

US-A-5,620,000 discloses a method for measuring the flow rate of a liquid, particularly of blood, in which the frequency shift of a laser beam reflected from the liquid is determined according to the optical Doppler effect. This system is designed so as to enable reliable measurement of the flow rate of the flowing medium which is resolved both spatially and with respect to time. At each scanning point a number of N measured values is collected corresponding to the reflected light, N being a whole number equal to or larger than 2; the Doppler shift is calculated from the time variation of the thus measured intensity of the reflected light at the respective scanning points, and the flow rate is determined from the Doppler shift at each point of the scanning pattern.

It is believed that all of the known prior art light scattering systems have had one or more disadvantages, including an inability to measure the concentration of relatively small particles in the presence of relatively large particles. Accordingly, there is a need for improved measurement systems and methods for determining component particle concentrations in a liquid, such as fat and casein concentrations in a dairy product.

According to one aspect of the present invention, there is provided a method for measuring casein concentration in a dairy product, characterised by the steps of generating a polarized light beam having a direction of polarization; placing a sample of the dairy product in a scattering cell that is positioned in said light beam, said scattering cell having a first window and a second window, wherein said light beam is incident on the second window after passing through the sample of the dairy product, wherein a normal to an exterior surface of the second window is at or near Brewster's angle with respect to said light beam and wherein the direction of polarization of said light beam is parallel to a plane defined by said light beam and the normal to the exterior surface of the second window; and detecting scattered light from casein particles in the dairy product at an angle in a range of about 130° to about 160° with respect to said light beam and generating a detector signal that is representative of casein concentration in the sample of the dairy product.

According to another aspect of the present invention, there is provided apparatus for measuring casein concentration in a dairy product, characterised by a light source for generating a polarized light beam having a direction of polarization; a scattering cell, positioned to receive said light beam, for receiving a sample of said dairy product, said scattering cell having a first window and a second window, wherein the second window is farther from said light source than is the first window, wherein a normal to an exterior surface of the second window is at or near Brewster's angle with respect to said light beam and wherein the direction of polarization of said light beam is parallel to a plane defined by said light beam and the normal to the exterior surface of the second window; and a light detector which is positioned at an angle in a range of about 130° to about 160° with respect to said light beam, and which serves to detect scattered light from casein of said dairy product and to generate a detector signal that is representative of concentration of said casein in said sample.

Owing to the invention, it is possible to provide a good system for the measurement of the concentration of casein in a dairy product, for example milk.

In a preferred embodiment, apparatus for measuring casein concentration in a dairy product comprises a light source for generating a polarized light beam having a direction of polarization, a scattering cell, positioned in the light beam, for receiving a sample of the dairy product, a first light detector positioned at a first angle in a range of about 5° to about 45° with respect to the light beam and a second light detector positioned at a second angle in a range of about 130° to about 160° with respect to the light beam. The scattering cell has a first window and a second window, wherein the second window is farther from the light source than the first window, wherein a normal to an exterior surface of the second window is at or near Brewster's angle with respect to the light beam and wherein the direction of polarization of the light beam is parallel to a plane defined by the light beam and the normal to the exterior surface of the second window. The first light detector detects scattered light from a first component of the dairy product having relatively large particle sizes and generates a first detector signal that is representative of concentration of the first component in the sample of the dairy product. The second light detector detects scattered light from the casein component of the dairy product having relatively small particle sizes and generates a second detector signal that is representative of concentration of the casein component in the sample of the dairy product. The first component may comprise fat.

In a preferred embodiment, the first angle is about 40° and the second angle is about 140°. Because of refraction effects in the first and second windows, the actual scattering angles differ from the laboratory angles which define the positions of the light detectors, as described below. The light source may comprise a laser and a polarizing device.

In the preferred embodiment, a method for measuring casein concentration in a dairy product comprises the steps of generating a polarized light beam having a direction of polarization, placing a sample of the dairy product in a scattering cell that is positioned in the light beam, detecting scattered light from a first component of the dairy product at a first angle in a range of about 5° to about 45° with respect to the light beam and generating a first detector signal, and detecting scattered light from casein particles

in the dairy product at a second angle in a range of about 130° to about 160° with respect to the light beam and generating a second detector signal. The scattering cell has a first window and a second window, the light beam is incident on the second window after passing through the sample of the dairy product, a normal to an exterior surface of the second window is at or near Brewster's angle with respect to the light beam, and the direction of polarization of the light beam is parallel to a plane defined by the light beam and the normal to the exterior surface of the second window. The first detector signal is representative of concentration of the first component in the sample of the dairy product, and the second detector signal is representative of casein concentration in the sample of the dairy product.

In order that the invention may be clearly and completely disclosed, reference will now be made, by way of example, to the accompanying drawings, in which:-
Fig. 1 is a graph of scattered light intensity as a function of scattering angle for relatively large particles and relatively small particles;
Fig. 2 is a simplified schematic representation of a measurement system in accordance with an embodiment of the invention;
Fig. 3 is a schematic representation of a light scattering system, illustrating a difficulty in light scattering measurement of fat and casein in milk;
Fig. 4 is a top schematic view of an embodiment of a measurement system in accordance with the invention;
Fig. 5A is a partial side view of the measurement system of Fig. 4;
Fig. 5B is a schematic side view of a first embodiment of the second window of the scattering cell, illustrating the orientation of the second window with respect to the light beam;
Fig. 5C is a schematic side view of a second embodiment of the second window of the scattering cell, illustrating the orientation of the second window with respect to the light beam;
Fig. 6 is a cross-sectional side view of an implementation of a scattering cell utilized in the measurement system of Figs. 4 and 5;
Fig. 7 is a block diagram that illustrates an application of the system for measuring component concentration in dairy products in an embodiment in accordance with the invention;
Fig. 8 is a top schematic view of a system for measuring component concentration in a liquid containing relatively large particles and relatively small particles;
Fig. 9 is a graph of sample composition wherein casein concentration in percent is plotted on the vertical axis and fat concentration in percent is plotted on the horizontal axis;
Fig. 10 is a graph that compares fat concentration in percent determined from light scattering measurements, plotted on the vertical axis, to fat concentration in percent determined from dilution, plotted on the horizontal axis; and
Fig. 11 is a graph that compares casein concentration in percent determined from light scattering measurement, plotted on the vertical axis, to casein concentration in percent determined from dilution, plotted on the horizontal axis.

A measurement system in accordance with the present invention exploits the way in which the physical properties of different components in a sample affect light scattered by the sample. For example, fat and casein in milk have different size distributions. Fat globule size distribution in raw bovine milk ranges from about 0.2 to 30 micrometers in diameter. After homogenization, the fat globule size is reduced so that most fat globules are under 3 micrometers in diameter, yielding a size distribution from about 0.2 to 3 micrometers. Casein occurs naturally in micellular form in the size range from 10 to 200 nanometers (0.01 to 0.2 micrometers) in diameter.

The intensity of scattered light is a function of particle size, light wavelength and polarization direction, scattering angle and sample concentration. The scattered intensity at a small angle is dependent almost entirely on the concentration of the large fat globules in the milk sample, and the scattered intensity at a large angle is dependent primarily on the concentration of the smaller casein micells.

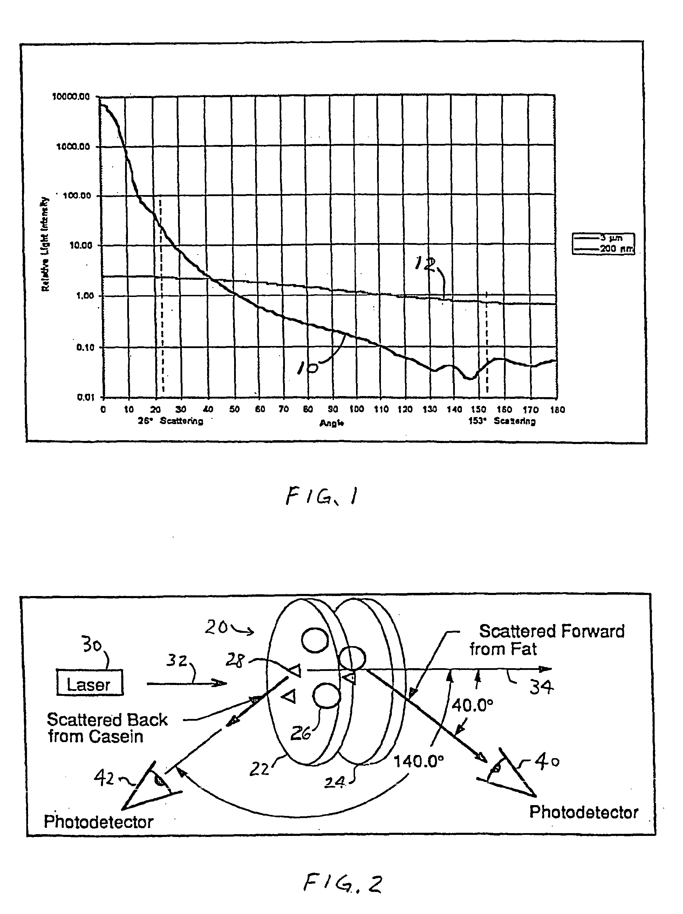

Fig. 1 shows the relative intensity of scattered light as a function of actual scattering angle for relatively large particles (3 micrometers) and relatively small particles (200 nanometers). Curve 10 represents the scattered light intensity from 3 micrometer particles, and curve 12 represents the scattered light intensity from 200 nanometer particles at a light wavelength of 800 nanometers. It may be observed that the relatively large particles exhibit a large variation, more than 100,000 to 1, in scattered light intensity as a function of scattering angle. By contrast, the relatively small particles exhibit a small variation, less than 10 to 1, in scattered light intensity as a function of scattering angle. For example, at an actual scattering angle of 26°, the intensity of light scattered from large fat particles substantially exceeds the intensity of light scattered from small casein particles. The reverse is true at an actual scattering angle of 153°. Thus, by separately measuring the scattered light at 40° and 140° as measured in the laboratory (actual scattering angles of 26.80° and 153 .20°, respectively), the fat and casein concentrations in the milk can be determined without the use of a chemical diluent. The actual scattering angle differs from the scattering angle measured in the laboratory (laboratory scattering angle) because of refraction effects in the scattering cell windows.

A simplified schematic diagram of an embodiment of a measurement system in accordance with the invention is shown in Fig. 2. The measurement system is configured for measuring fat and casein concentrations in a dairy product. A scattering cell 20, represented in Fig. 2 by a first window 22 and a second window 24, contains a sample of a dairy product, such as diluted milk, for measurement. The dairy product contains fat particles 26 and casein particles 28. The sample is located between windows 22 and 24 and flows continuously through scattering cell 20. Scattering cell 20 is discussed in more detail below. A laser 30 directs a laser beam 32 along an optical axis 34 through scattering cell 20. Laser beam 32 has a direction of polarization perpendicular to the plane of Fig. 2. As described in detail below, windows 22 and 24 are oriented at Brewster's angle with respect to the polarized laser beam 32. The laser beam 32 passes through first window 22, through the sample, and through second window 24. As used herein, the "first" window refers to the scattering cell window that is closest to laser 30 or other light source, and the "second" window refers to the scattering cell window that is farthest from laser 30 or other light source. Thus, laser beam 32 is incident on the second window after passing through the liquid sample.

The laser beam 32 passing through scattering cell 20 is scattered by components of the dairy product sample. The angular distribution of the scattered light depends upon the properties of the particles in the sample, including their concentrations. In the embodiment of Fig. 2, a first photodetector 40 is located at an angle of 40° with respect to optical axis 32, and a second photodetector 42 is located at an angle of 140° with respect to optical axis 32. First photodetector 40 detects scattered light at an angle of 40° and provides a detector signal that is representative of fat concentration in the liquid sample. Second photodetector 42 detects scattered light at an angle of 140° and provides a detector signal that is representative of casein concentration in the liquid sample. As shown in Fig. 1, the scattered light intensity from fat is significantly greater than the scattered light intensity from casein at small angles, and the scattered light intensity from casein is significantly larger than the scattered light intensity from fat at large scattering angles. Accordingly, the measurement system of Fig. 2 provides measurements of the concentrations of fat and casein in the dairy product.

The separation between fat and casein measurements is incomplete. Thus, the detector signal from photodetector 40 at an angle of 40° contains a predominant component from fat and a lesser component from casein. Similarly, the detector signal from photodector 42 at an angle of 140° contains a predominant component from casein and a lesser component from fat. This is apparent from Fig. 1. The unprocessed detector signals therefore provide approximations to the fat and casein concentrations. As described below, more accurate values of fat and casein concentration can be obtained by processing the detector signals.

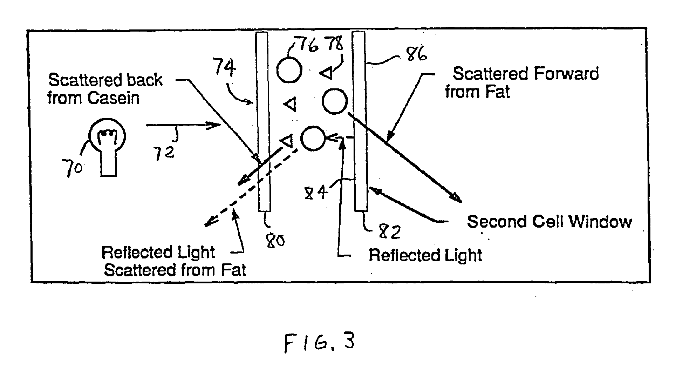

Generally, it is not possible to measure scattered light from small particles, such as casein, at large angles in the presence of large particles, such as fat, using prior art scattering cells. The reason is described with reference to Fig. 3, which is a schematic representation of a light scattering measurement system utilizing a conventional scattering cell. Light from a light source 70 is directed along an optical axis 72 through a scattering cell 74 containing a dairy product, including relatively large fat particles 76 and relatively small casein particles 78. A first window 80 and a second window 82 of scattering cell 74 have surfaces oriented perpendicular to optical axis 72. In the system of Fig. 3, the light beam from light source 70 is partially reflected by glass-liquid interface 84 and glass-air interface 86 of second window 82. The reflected light is scattered in a forward direction by fat particles 76 in the same direction as the light scattered by casein particles 78 in a backward direction from the main light beam. The reflected light scattered by fat particles 76 is more intense than the light scattered by casein particles 78 from the main light beam. For this reason, casein concentration cannot be measured in the system of Fig. 3.

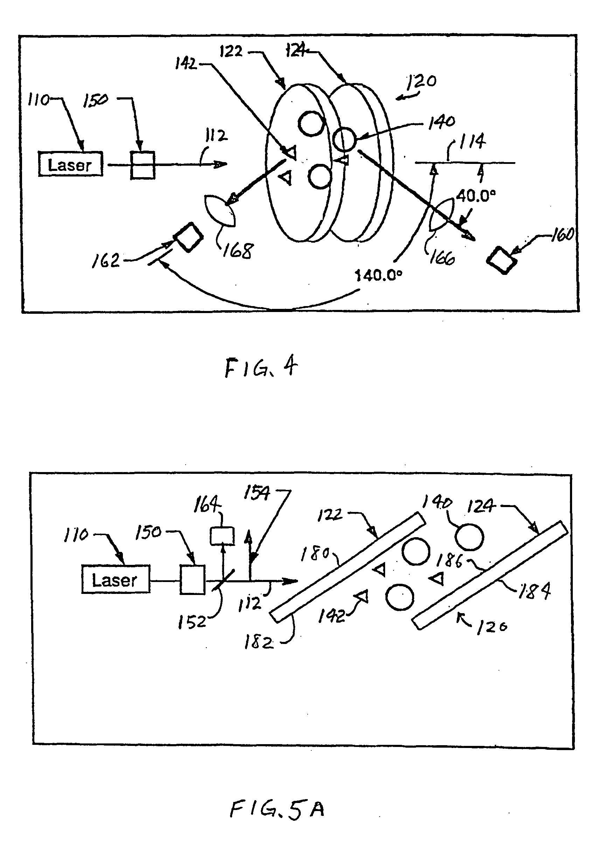

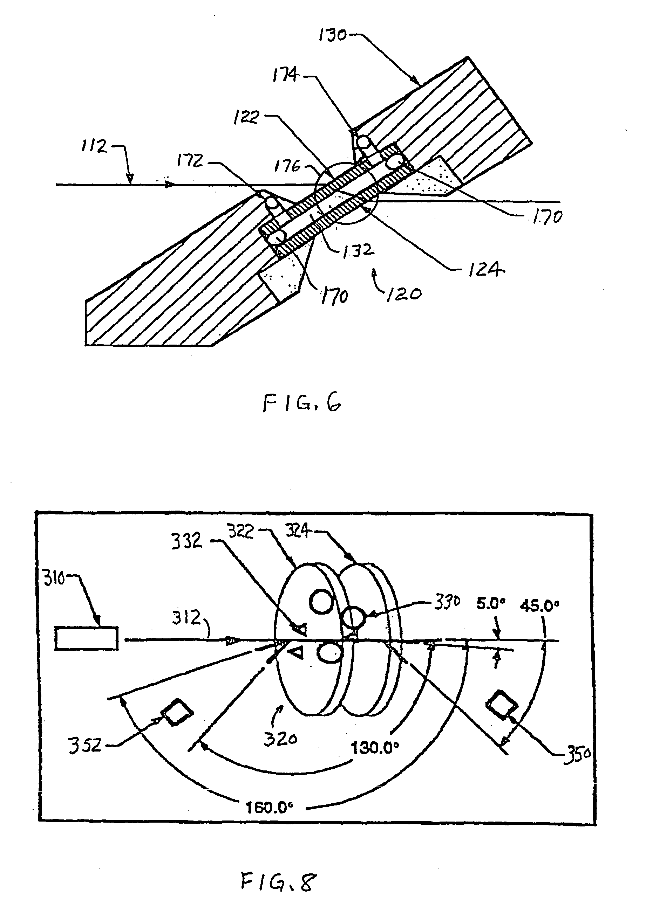

An embodiment of a light scattering measurement system in accordance with the invention is shown in Figs. 4, 5A and 6. Fig. 4 is a schematic top view of the measurement system, and Fig. 5A is a schematic partial side view of the measurement system. Fig. 6 is a cross-sectional side view of an implementation of the scattering cell. Like elements in Figs. 4, 5A and 6 have the same reference numerals. It will be understood that the orientation of the system can be changed and that the top and side designations are arbitrary.

A laser 110 directs a laser beam 112 along an optical axis 114 through a scattering cell 120. Scattering cell 120 includes a first window 122, a second window 124 and a housing 130 (Fig. 6). Housing 130 encloses a scattering cell volume 132 between first window 122 and second window 124. As indicated above, first window 122 is closest to laser 110 and second window 124 is farthest from laser 110. Scattering cell 120 contains a sample for measurement, including relatively large fat particles 140 and relatively small casein particles 142. As shown in Fig. 5A, laser beam 112 is directed through a polarizing device 150 and a beam splitter 152 before reaching scattering cell 120. As shown in Fig. 5A, polarizing device 150 causes laser beam 112 to have a vertical direction of polarization 154, so that a polarized light beam is incident on scattering cell 120. A first photodetector 160 is oriented at an angle of 40° in a horizontal plane with respect to laser beam 112. A second photodetector 162 is oriented at an angle of 140° in the horizontal plane with respect to laser beam 112. A third photodetector 164 is oriented at 90° with respect to laser beam 112 for monitoring the light intensity emanating from beam splitter 152. Photodetector 164 functions as a reference detector to normalize fluctuations in light intensity generated by laser 110.

Preferably, laser beam 112 is in a wavelength range from about 780 nanometers to about 1100 nanometers for measuring fat and casein concentrations in dairy products. In one example of the measurement system, laser 110 is a 780 nanometer, 2.5 milliwatt, polarized diode laser available from Melles-Griot. Although the laser provides a polarized laser beam, approximately 1 % of the light has the wrong polarization and a separate polarizing device may be required.

Photodetectors 160, 162 and 164 may be type S2386-8K available from Hamamatsu. Photodetectors 160 and 162 are not limited to angles of 40° and 140°, respectively, with respect to laser beam 112. More generally, photodetector 160 may be located at a laboratory angle in a range of about 5° to 45° for measuring scattered light from fat particles, and photodetector 162 maybe located at a laboratory angle in a range of about 130° to 160° for measuring scattered light from casein particles. As described above, photodetectors 160 and 162 do not provide complete separation between fat scattering measurements and casein scattering measurements. Lenses 166 and 168 form images of the scattering region on the active areas of photodetectors 160 and 162, respectively. The active areas of the preferred photodiodes are 5.8 x 5.8 millimeters.

As shown in Fig. 6, scattering cell 120 includes first window 122, second window 124 and housing 130, which enclose cell volume 132. The cell volume 132 thickness between first window 122 and second window 124 is defined by spacers 170. The liquid being measured flows through cell volume 132 from an inlet port 172 to an outlet port 174. According to a further feature, the scattering cell 120 may include a window, shown schematically at 176, positioned at 90° with respect to light beam 112, to permit measurement of 90° light scattering.

In one example of scattering cell 120, first window 122 and second window 124 each have a thickness of 0.15 inch and are fabricated of fused silica. Cell volume 132 is a circular disk having a diameter of 1.10 inch. The cell volume thickness between windows 122 and 124, defined by spacers 170, is 0.015 inch. Inlet port 172 and outlet port 174 have diameters of 0.10 inch. It will be understood that these scattering cell parameters and the system components identified above are given by way of example only and are not limiting as to the scope of the invention.

As indicated above, light scattered from relatively large particles at small angles is much more intense than light scattered at large angles. For example, a particle 3 micrometers in diameter scatters light about 1,000 times more intensely at an angle of 20° than at an angle of 160°. For this reason, it is not possible to make accurate measurements of small particles at large scattering angles using conventional light scattering cells when large particles are present. The reason is that, even if the surfaces of the cell windows have anti-reflection coatings, a small amount of the main light beam is reflected at the glass-air and glass-liquid interfaces. The light detected at a large scattering angle is made up of two components: (1) light scattered at the large angle from the incident light beam, and (2) light scattered at a small angle from the reflected beam parallel to the first component, as illustrated in Fig. 3. Even if the total reflected light intensity is only 0.5% (the reflection from a glass-water interface is about 0.3%), the undesired scattered light in the above example is five times as intense as the desired scattered light in a measurement at a scattering angle of 160°.

The reflected light may be eliminated or nearly eliminated by using a polarized light source and tilting at least the second scattering cell window, the window that is farthest from the light source, at or near Brewster's angle with respect to the polarized light beam. As known in the art, Brewster's angle is the angle between a light beam incident on an interface between two materials and a normal to the interface at which no reflection occurs. Brewster's angle, θ_{B}, is defined as tan θ_{B} = n₂/n₁, where n₁ and n₂ are the indices of refraction of the materials on opposite sides of the interface, with n₁ corresponding to the medium through which the light beam is incident on the interface.

As illustrated in Fig. 5A, first window 122 has an exterior surface 180 and an interior surface 182; and second window 124 has an exterior surface 184 and an interior surface 186. In general, reflections from the surfaces 180 and 182 of first window 122 do not cause a problem, because the reflections do not pass through the liquid sample in scattering cell 120 and thus are not scattered toward photodetector 162. With respect to second window 124, light is reflected from interior surface 186 and exterior surface 184, when these surfaces are not oriented at Brewster's angle. Typically, the reflections from exterior surface 184 are more intense than the reflections from interior surface 186, because the indices of refraction at the glass-air interface (exterior surface 184) differ by more than the indices of refraction at the glass-liquid interface (interior surface 186).

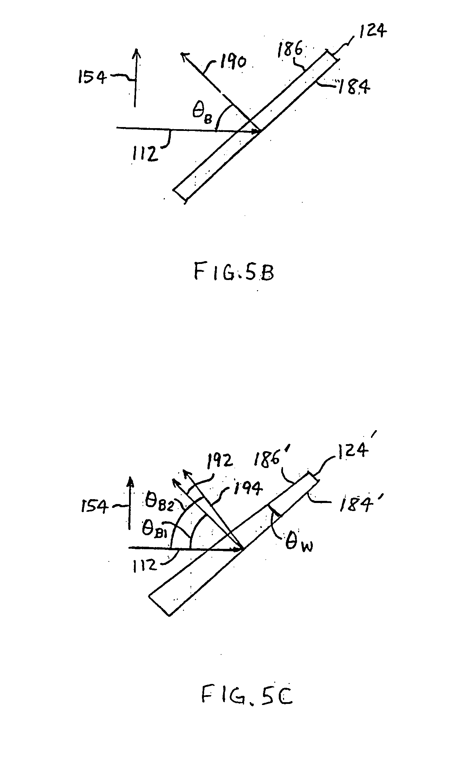

In a first embodiment, the interior surface 186 and the exterior surface 184 of second window 124 are parallel, and window 124 is mounted at or near Brewster's angle with respect to light beam 112 for the glass-air interface of surface 184. The orientation of window 124 with respect to light beam 112 is illustrated in the schematic diagram of Fig. 5B. Light beam 112 is incident on surface 184 at Brewster's angle θ_{B} with respect to a normal 190 to exterior surface 184. Brewster's angle θ_{B} is determined as described above with respect to exterior surface 184, a glass-air interface. In this embodiment, second window 124 is preferably tilted at an angle θ_{B} of 55.463°, Brewster's angle for a fused silica-air interface. Second window 124 is further oriented such that the direction of polarization 154 of light beam 112 is parallel to a plane defined by light beam 112 and normal 190 to exterior surface 184 of second window 124. This configuration ensures little or no reflection of light beam 112 from exterior surface 184. For convenience, first window 122 may be mounted parallel to second window 124. However, first window 122 may have other orientations within the scope of the invention.

Because exterior surface 184 is a glass-air interface and interior surface 186 is a glass-liquid interface, Brewster's angle is different for the two surfaces. Thus, when exterior surface 184 is oriented at or near Brewster's angle with respect to light beam 112, interior surface 186 is not oriented at Brewster's angle with respect to light beam 112, and a small fraction of light beam 112 may be reflected from interior surface 186. However, it has been found that the reflections from the glass-liquid interface of surface 186 are small and do not interfere to a significant degree with casein concentration measurements at a scattering angle of 140°.

In a second embodiment, both exterior surface 184 and interior surface 186 of second window 124 are oriented at or near Brewster's angle for the respective interfaces. As noted above, Brewster's angle is different for the interfaces at surfaces 184 and 186. In order to insure that each surface is oriented at the respective Brewster's angle, second window 124 is fabricated with a wedge shape. A wedge-shaped second window 124' is shown in Fig. 5C. Exterior surface 184' and interior surface 186' of second window 124' diverge at a wedge angle θ_{w} that is equal to the difference in Brewster's angle for the interfaces at surfaces 184' and 186'. In the example of a fused silica window with water as the fluid, the wedge angle θ_{w} is 7.997°. The wedge angle θ_{w} is exaggerated in Fig. 5C for illustrative purposes. Thus, second window 124' is oriented such that a normal 192 to exterior surface 184' is at or near Brewster's angle θ_{B1} with respect to light beam 112 for exterior surface 184', and a normal 194 to interior surface 186' is at or near Brewster's angle θ_{B2} with respect to light beam 112 for interior surface 186'. As in the first embodiment, the direction of polarization 154 of light beam 112 is parallel to a plane defined by light beam 112 and the normal 192 to exterior surface 184' of second window 124'. Using the wedge-shaped window 124' with both surfaces oriented at Brewster's angle, light beam 112 is not reflected from interior surface 186' or exterior surface 184'. In the second embodiment, first window 122 may be wedge-shaped for convenience of manufacturing or may have parallel surfaces. In general, it is not necessary to orient first window 122 at Brewster's angle. However, the use of two wedge-shaped windows may facilitate manufacturing of the scattering cell.

By tilting the windows of the sample cell 120 at or near Brewster's angle, as described above, little or no light is reflected back into the cell. The scattered light from the casein in the sample is measured at 140° without interference and is proportional to the casein concentration. A signal proportional to fat concentration is measured at 40°. Thus, referring again to Fig. 4, photodetector 160 provides a detector signal that is representative of fat concentration in the sample, and photodetector 162 provides a detector signal that is representative of casein concentration. As described above, photodetectors 160 and 162 do not provide complete separation between fat scattering measurements and casein scattering measurements.

Data analysis to determine fat and casein concentrations depends on the sample concentration. Sufficiently dilute samples generate single scattering (light rays scatter only once before exiting the cell) and produce a light intensity signal which increases linearly with component concentration. Fat and casein sample concentration are each related to measured light intensity at 40° and 140°, respectively, by Y = mX + b, where X is the measured light intensity, and m and b are calibration coefficients for the specific system.

More concentrated samples generate multiple scattering, wherein light rays scatter from more than one particle before exiting the cell. Component concentration is given by a polynomial equation in which the higher order terms account for multiply scattered light. Fat and casein concentrations may each be calculated from measured light intensity at 40° and 140°, respectively, by Y = aX + bX² + c, where X is the measured light intensity, and a, b, and c are calibration coefficients for the specific system.

Since fat contributes to some of the scattered light measured at 140° due to both multiple and single scattering, and casein contributes to some of the scattered light measured at 40° for the same reasons, more accurate results may be calculated using polynomial expressions of multiple variables. For example, fat concentration may be calculated from F = aX + bX² + cZ + dZ² + e, where X is the light intensity measured at 40°, Z is the light intensity measured at 140°, and a, b, c, d, and e, are calibration coefficients for the specific system. Casein concentration may be calculated from C = fX + gX² + hZ + iZ² + j, where X is the light intensity measured at 40°, Z is the light intensity measured at 140°, and f, g, h, i and j are calibration coefficients for the specific system. Other mathematical forms which account for the non-linearity of specific implementations will yield optimum results for those implementations. The calibration coefficients may be determined experimentally for a particular system.

The calibration coefficients may be determined by the following steps.
1. Obtain two samples with known casein and fat concentrations, one with high fat concentration and low casein concentration, and the other with low fat concentration and high casein concentration.
2. Make a number of mixtures of the two samples to obtain second samples with a range of concentrations of fat and casein.
3. Make light scattering measurements on the second samples.
4. Use standard curve fitting techniques to determine the calibration coefficients from the results of the scattering measurements.

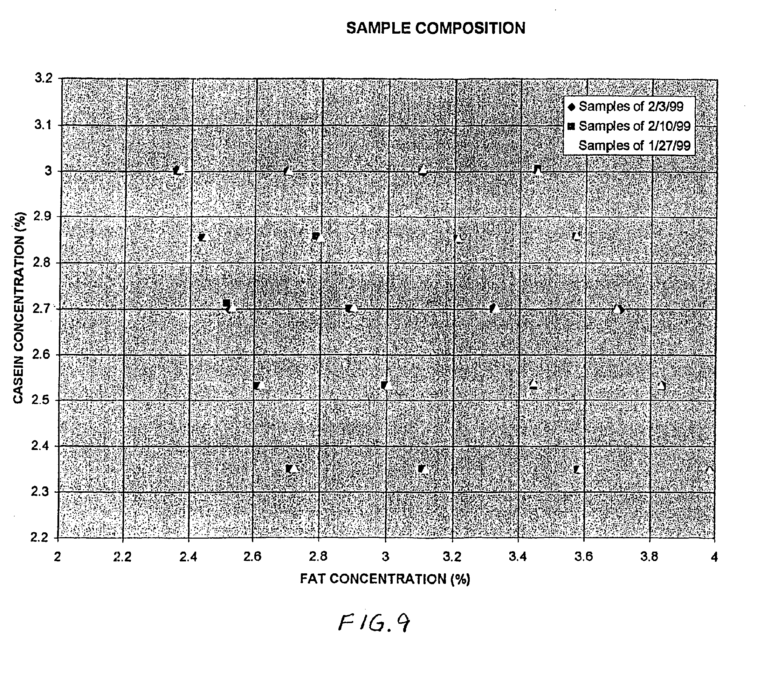

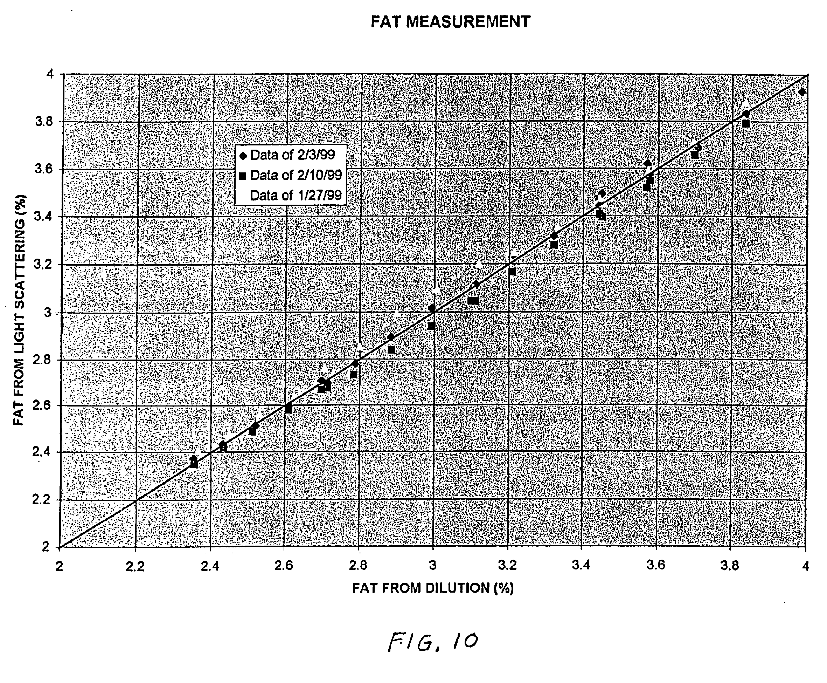

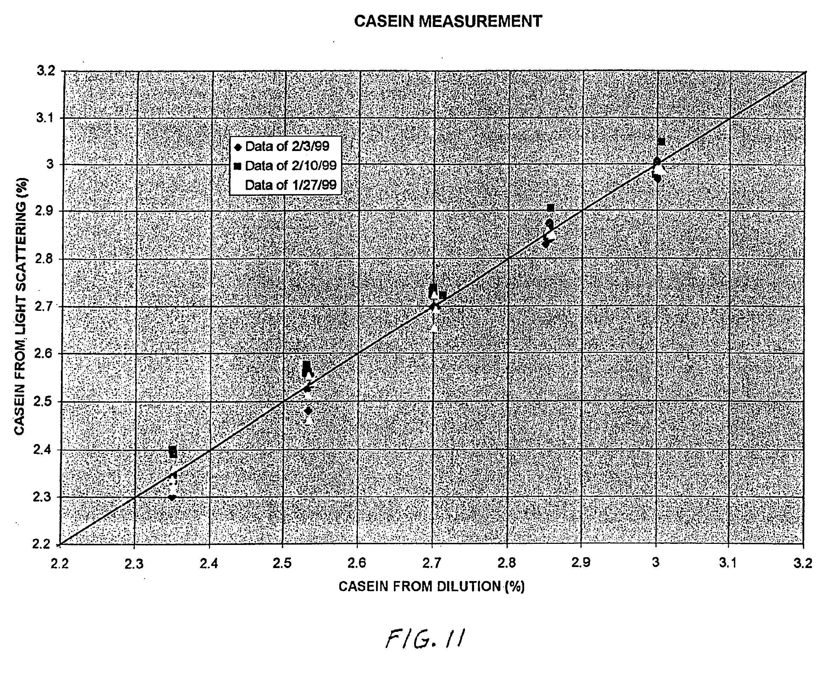

This process is illustrated in Figs. 9-11. Fig. 9 shows the casein and fat concentrations for twenty different samples. The casein concentration in percent is plotted on the vertical axis, and the fat concentration in percent is plotted on the horizontal axis. Fig. 10 shows the fat concentrations determined from light scattering measurements compared to fat concentrations determined from dilutions. In Fig. 10, fat concentration in percent determined from light scattering measurements is plotted on the vertical axis, and fat concentration in percent determined from dilution is plotted on the horizontal axis. Fig. 11 shows the casein concentrations determined from light scattering measurements compared to casein concentrations determined from dilutions. In Fig. 11, casein concentration in percent determined from light scattering measurements is plotted on the vertical axis, and casein concentration in percent determined from dilution is plotted on the horizontal axis.

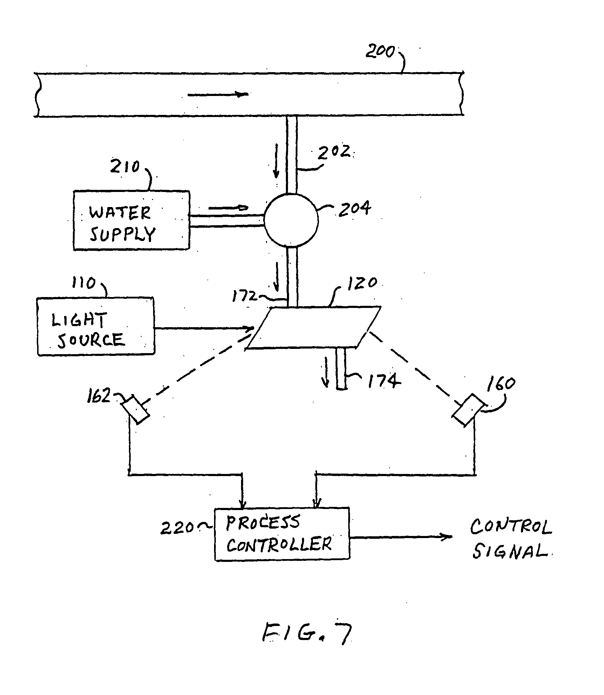

A simplified block diagram of an example of an application of the measurement system of the present invention in the dairy processing industry is shown in Fig. 7. Fig. 7 may represent a part of a cheese making facility. A system of the type shown in Figs. 4-6 and described above is utilized. Like elements in Figs. 4-7 have the same reference numerals. A conduit 200 carries a dairy product, such as milk, for use in the process. A sample of the dairy product is diverted through a smaller conduit 202 to a first inlet of a mixing valve 204. A water supply 210 provides water to a second inlet of mixing valve 204. Mixing valve 204 provides the ability to dilute the dairy product with water to facilitate light scattering measurements. In a preferred embodiment, milk is diluted with eight parts water to one part milk to provide a suitable sample for light scattering measurements. However, it will be understood that different dilution ratios may be utilized within the scope of the invention.

The diluted milk is provided as a liquid sample through inlet port 172 of scattering cell 120. The liquid sample passes through scattering cell 120 and outlet port 174 and is discarded after measurement. Light scattering measurements of fat concentration are made by photodetector 160 at a scattering angle of 40°, and light scattering measurements of casein concentration are made by photodetector 162 at a scattering angle of 140°, as described above. The detector signals are supplied to a process controller 220, which may comprise a personal computer (PC) or other computer or dedicated process controller. The process controller 220 analyzes the measured fat and casein concentrations and generates a control signal based on the fat and casein concentrations. In one example, the control signal varies the fat concentration in the dairy product passing through conduit 200 to achieve a desired casein to fat ratio. It will be understood that different control functions may be based on the measured fat and casein concentrations.

A measurement system for determining fat and casein concentrations in a dairy product is described above in connection with Figs. 2, 4, 5A, 6 and 7. However, a system of the type described above may be utilized more generally for measuring particle concentrations in a liquid containing relatively large particles and relatively small particles.

A schematic top view of a measurement system in accordance with an embodiment of the invention is shown in Fig. 8. A light source 310 directs a polarized light beam 312 through a scattering cell 320. The light source 310 may be a laser or any other light source that produces a collimated beam. If necessary, the light source includes a polarizing device, such as a polarizing filter, to generate polarized light beam 312. Scattering cell 320 is defined by a first window 322 and a second window 324. A housing (not shown) encloses a cell volume between windows 322 and 324. At least the exterior surface of second window 324 is oriented at or near Brewster's angle with respect to light beam 312, as described above. Scattering cell 320 contains a liquid sample, including relatively large particles 330 and relatively small particles 332.

A first photodetector 350 at an angle of less than 90° with respect to light beam 312 detects scattered light from large particles 330, and a second photodetector 352 at an angle greater than 90° detects scattered light from small particles 332. As described above, photodetectors 350 and 352 do not provide complete separation between fat scattering measurements and casein scattering measurements. Photodetectors 350 and 352 are located in a horizontal plane that passes through light beam 312, where light beam 312 has a vertical direction of polarization. As indicated above, the orientation of the entire measurement system may be varied within the scope of the invention. The range of scattering angles available for measurement is limited because the scattered light must pass either through the first window 322 for large scattering angles or the second window 324 for small scattering angles. The accessible scattering angle ranges are typically 5° to 45° in the forward direction and 130° to 160° in the backward direction, as measured in the laboratory. Thus, detector 350 is positioned within a range of 5° to 45° with respect to light beam 312, and photodetector 352 is positioned within a range of 130° to 160° with respect to light beam 312. Because of refraction effects in windows 322 and 324, the actual scattering angles are 3.44° to 29.92°, corresponding to laboratory angles of 5° to 45°, and 147.03° to 166.33°, corresponding to laboratory angles of 130° to 160°.

In the measurement systems described above, measurements of light scattering from relatively large particles, such as fat particles, and measurements of light scattering from relatively small particles, such as casein particles, may be made simultaneously or at different times. Furthermore, the measurement systems may be configured for measuring one of the particle types. In the measurement system of Fig. 4, photodetector 162 may be utilized for measurements of casein concentration, and photodetector 160 may be omitted if measurement of fat concentration is not required. Alternatively, in an example which is not part of the invention photodetector 160 may be utilized for measurements of fat concentration, and photodetector 162 may be omitted if measurement of casein concentration is not required. Similarly, in the measurement system of Fig. 8, photodetector 350 or photodetector 352 may be omitted when measurement of the respective particle concentration is not required, however, it is not part of the invention that measurement of the casein concentration is not required. In each case, the disclosed measurement system provides the capability to measure particle concentration in a liquid containing both relatively large particles and relatively small particles.

While there have been shown and described what are at present considered the preferred embodiments of the present invention, it will be obvious to those skilled in the art that various changes and modifications may be made therein without departing from the scope of the invention as defined by the appended claims.

## Claims

1. A method for measuring casein concentration in a dairy product, **characterised by** the steps of generating a polarized light beam (32) having a direction of polarization; placing a sample of the dairy product in a scattering cell (20) that is positioned in said light beam (32), said scattering cell having a first window (22) and a second window (24), wherein said light beam (32) is incident on the second window (24) after passing through the sample of the dairy product, wherein a normal to an exterior surface of the second window (24) is at or near Brewster's angle with respect to said light beam (32) and wherein the direction of polarization of said light beam (32) is parallel to a plane defined by said light beam (32) and the normal to the exterior surface of the second window (24); and detecting scattered light from casein particles in the dairy product at an angle in a range of about 130° to about 160° with respect to said light beam (32) and generating a detector signal that is representative of casein concentration in the sample of the dairy product.

2. A method according to claim 1, wherein said angle is about 140°.

3. A method according to claim 1 or 2 and also for measuring fat concentration in said dairy product, and further comprising detecting scattered light from fat particles in the dairy product at an angle in a range of about 5° to about 45° with respect to said light beam and generating a second detector signal that is representative of fat concentration in said sample.

4. A method according to claim 3, wherein the latter angle is about 40°.

5. A method according to claim 3 or 4 and further comprising the step of generating a signal representative of a casein to fat ratio in said sample in response to the first-mentioned and said second detector signals.

6. A method according to any preceding claim and further comprising the step of diluting the dairy product with water to provide said sample.

7. A method according to any preceding claim, wherein said light beam (32) has a wavelength in a range of about 780 to 1100 nanometers.

8. Apparatus for measuring casein concentration in a dairy product **characterised by**: a light source (30) for generating a polarized light beam (32) having a direction of polarization; a scattering cell (20), positioned to receive said light beam (32), for receiving a sample of said dairy product, said scattering cell (20) having a first window (22) and a second window (24), wherein the second window (24) is farther from said light source (30) than is the first window (22), wherein a normal to an exterior surface of the second window (24) is at or near Brewster's angle with respect to said light beam (32) and wherein the direction of polarization of said light beam (32) is parallel to a plane defined by said light beam (32) and the normal to the exterior surface of the second window (24); and a light detector (42) which is positioned at an angle in a range of about 130° to about 160° with respect to said light beam (32), and which serves to detect scattered light from casein of said dairy product and to generate a detector signal that is representative of concentration of said casein in said sample.

9. Apparatus according to claim 8, wherein said angle is about 140°.

10. Apparatus according to claim 8 or 9 and further comprising a data analyzer (220), responsive to the detector signal, for determining casein concentration in said sample.

11. Apparatus according to claim 8 or 9 and further comprising: another light detector (40), positioned at another angle in a range of about 5° to about 45°with respect to said light beam (32) for detecting scattered light from fat particles of said dairy product and generating a second detector signal that is representative of concentration of said fat particles in said sample.

12. Apparatus according to claim 11, wherein said other angle is about 40°.

13. Apparatus according to claim 11 or 12 and further comprising a data analyzer (220), responsive to the first-mentioned and said second detector signals, for generating a signal representative of a casein to fat ratio in said sample.

14. Apparatus according to any one of claims 8 to 13, wherein said light source (30) comprises a laser (110) and a polarizing device (150).

15. Apparatus according to any one of claims 8 to 13, wherein said light source (110) comprises a laser diode (110) which serves to generate said polarized light beam (32).

16. Apparatus according to any one of claims 8 to 15, wherein said light source (30) serves to emit said light beam (32) with a wavelength in a range of about 780 to 1100 nanomerters.

17. Apparatus according to any one of claims 8 to 16, wherein the first window (22) and the second window (24) of said scattering cell (20) have parallel surfaces.

18. Apparatus according to any one of claims 8 to 17, wherein said scattering cell (20) includes an inlet port and an outlet port to permit said sample to flow through the scattering cell (20).

19. Apparatus according to any one of claims 8 to 18, wherein an interior surface of the second window (24) is parallel to the exterior surface of the second window (24).

20. Apparatus according to any one of claims 8 to 18, wherein a normal to an interior surface (186') of the second window (124') for contacting said sample is at or near Brewster's angle with respect to said light beam (112), and wherein the second window (124') is wedge-shaped.

21. Apparatus according to any one of claims 8 to 20 and further comprising means for diluting said dairy product with water to provide said sample.

## Patentansprüche

1. Verfahren zum Messen der Kaseinkonzentration in einem Milchprodukt, welches durch die folgenden Schritte **gekennzeichnet** ist: Erzeugen eines polarisierten Lichtstrahls (32), der eine Polarisierungsrichtung aufweist; Anordnen einer Probe eines Milchprodukts in einer Streuungszelle (20), die in dem besagten Lichtstrahl (32) angeordnet ist, wobei die Streuungszelle ein erstes Fenster (22) und ein zweites Fenster (24) aufweist, und der Lichtstrahl (32) auf das zweite Fenster (24) einfällt, nachdem er die Milchproduktprobe durchdrungen hat, wobei eine Normale auf eine Außenoberfläche des zweiten Fensters (24) hinsichtlich des Lichtstrahls (32) im oder nahe dem Brewster-Winkel liegt, und wobei die Polarisierungsrichtung des Lichtstrahls (32) parallel zu einer Ebene ist, die durch den Lichtstrahl (32) und die Normale auf die Außenoberfläche des zweiten Fensters (24) definiert ist; und Detektieren des von Kaseinpartikeln in dem Milchprodukt gestreuten Lichts unter einem Winkel im Bereich von etwa 130° bis etwa 160° in Bezug auf den Lichtstrahl (32) und Erzeugen eines Detektorsignals, das für die Kaseinkonzentration in der Milchproduktprobe repräsentativ ist.

2. Verfahren nach Anspruch 1, wobei der Winkel etwa 140° beträgt.

3. Verfahren nach Anspruch 1 oder 2, welches auch der Messung der Fettkonzentration in dem Milchprodukt dient, und weiters den Schritt aufweist, das von Fettpartikeln in dem Milchprodukt gestreute Licht unter einem Winkel im Bereich von etwa 5° bis etwa 45° in Bezug auf den Lichtstrahl zu detektieren und ein zweites Detektorsignal zu erzeugen, das für die Fettkonzentration in der Probe repräsentativ ist.

4. Verfahren nach Anspruch 3, wobei der letztgenannte Winkel etwa 40° beträgt.

5. Verfahren nach Anspruch 3 oder 4, welches weiters den Schritt aufweist, in Reaktion auf das zuerst genannte und das zweite Detektorsignal ein Signal zu erzeugen, das für ein Verhältnis von Kasein zu Fett in der Probe repräsentativ ist.

6. Verfahren nach einem der vorhergehenden Ansprüche, welches weiters den Schritt aufweist, das Milchprodukt mit Wasser zu verdünnen, um die Probe bereitzustellen.

7. Verfahren nach einem der vorhergehenden Ansprüche, wobei der Lichtstrahl (32) eine Wellenlänge im Bereich von etwa 780 bis 1100 Nanometer aufweist.

8. Vorrichtung zum Messen der Kaseinkonzentration in einem Milchprodukt, **gekennzeichnet durch** eine Lichtquelle (30) zum Erzeugen eines polarisierten Lichtstrahls (32), der eine Polarisierungsrichtung aufweist; eine zum Empfang des Lichtstrahls (32) angeordnete Streuungszelle (20), zur Aufnahme einer Probe des Milchprodukts, wobei die Streuungszelle (20) ein erstes Fenster (22) und ein zweites Fenster (24) aufweist, wobei das zweite Fenster (24) weiter von der Lichtquelle (30) entfernt ist, als das erste Fenster (22), wobei eine Normale auf eine Außenoberfläche des zweiten Fensters (24) hinsichtlich des Lichtstrahls (32) im oder nahe dem Brewster-Winkel liegt, und wobei die Polarisierungsrichtung des Lichtstrahls (32) parallel zu einer Ebene ist, die **durch** den Lichtstrahl (32) und die Normale auf die Außenoberfläche des zweiten Fensters (24) definiert ist; und einen Lichtdetektor (42), der in einem Winkel im Bereich von etwa 130° bis etwa 160° in Bezug auf den Lichtstrahl (32) angeordnet ist, und der dazu dient, das von dem Kasein des Milchprodukts gestreute Licht zu detektieren und ein Detektorsignal zu erzeugen, das für die Kaseinkonzentration in der Milchproduktprobe repräsentativ ist.

9. Vorrichtung nach Anspruch 8, wobei der Winkel etwa 140° beträgt.

10. Vorrichtung nach Anspruch 8 oder 9, welche weiters eine Daten-Analyseeinheit (220) aufweist, die auf die Detektorsignale reagiert, um die Kaseinkonzentration in der Probe zu ermitteln.

11. Vorrichtung nach Anspruch 8 oder 9, welche weiters einen weiteren Lichtdetektor (40) aufweist, der unter einem anderen Winkel in einem Bereich von etwa 5° bis etwa 45° in Bezug auf den Lichtstrahl (32) angeordnet ist, um das von Fettpartikeln des Milchprodukts gestreute Licht zu detektieren und ein zweites Detektorsignal zu erzeugen, das für die Konzentration der Fettpartikel in der Probe repräsentativ ist.

12. Vorrichtung nach Anspruch 11, wobei der besagte andere Winkel etwa 40° beträgt.

13. Vorrichtung nach Anspruch 11 oder 12, welche weiters eine Daten-Analyseeinheit (220) aufweist, die auf die zuerst genannten und die zweiten Detektorsignale reagiert, um ein Signal zu erzeugen, das für ein Verhältnis von Kasein zu Fett in der Probe repräsentativ ist.

14. Vorrichtung nach einem der Ansprüche 8 bis 13, wobei die Lichtquelle (30) einen Laser (110) und eine Polarisierungseinheit (150) aufweist.

15. Vorrichtung nach einem der Ansprüche 8 bis 13, wobei die Lichtquelle (110) eine Laserdiode (110) aufweist, die dazu dient, den polarisierten Lichtstrahl (32) zu erzeugen.

16. Vorrichtung nach einem der Ansprüche 8 bis 15, wobei die Lichtquelle (30) dazu dient, den Lichtstrahl (32) mit einer Wellenlänge in einem Bereich von etwa 780 bis 1100 Nanometer abzugeben.

17. Vorrichtung nach einem der Ansprüche 8 bis 16, wobei das erste Fenster (22) und das zweite Fenster (24) der Streuungszelle (20) parallele Oberflächen aufweisen.

18. Vorrichtung nach einem der Ansprüche 8 bis 17, wobei die Streuungszelle (20) eine Einlassöffnung und eine Auslassöffnung aufweist, um der Probe zu ermöglichen, durch die Streuungszelle (20) hindurch zu fließen.

19. Vorrichtung nach einem der Ansprüche 8 bis 18, wobei eine Innenoberfläche des zweiten Fensters (24) zur Außenoberfläche des zweiten Fensters (24) parallel ist.

20. Vorrichtung nach einem der Ansprüche 8 bis 18, wobei eine Normale auf eine mit der Probe in Kontakt stehende Innenoberfläche (186') des zweiten Fensters (124') hinsichtlich des Lichtstrahls (112) im oder nahe dem Brewster-Winkel liegt, und wobei das zweite Fenster (124') eine Keilform aufweist.

21. Vorrichtung nach einem der Ansprüche 8 bis 20, welche weiters Hilfsmittel aufweist, um das Milchprodukt mit Wasser zu verdünnen, um die Probe bereitzustellen.

## Revendications

1. Procédé pour mesurer la concentration de caséine dans un produit laitier, **caractérisé par** les étapes consistant à : générer un faisceau de lumière polarisée (32) possédant une direction de polarisation ; placer un échantillon du produit laitier dans une cellule de diffusion (20) qui est disposée dans ledit faisceau de lumière (32), ladite cellule de diffusion possédant une première fenêtre (22) et une deuxième fenêtre (24), ledit faisceau de lumière (32) étant un faisceau incident qui tombe sur la deuxième fenêtre (24) après avoir traversé l'échantillon du produit laitier, une normale à la surface externe de la deuxième fenêtre (24) formant un angle de Brewster ou approximativement un angle de Brewster par rapport audit faisceau de lumière (32) et la direction de polarisation dudit faisceau de lumière (32) étant parallèle à un plan défini par ledit faisceau de lumière (32) et la normale à la surface externe de la deuxième fenêtre (24) ; et détecter la lumière diffusée par les particules de caséine dans le produit laitier à un angle dans la plage d'environ 130° à environ 160° par rapport audit faisceau de lumière (32) ; et générer un signal de détection qui est représentatif pour la concentration de caséine dans l'échantillon du produit laitier.

2. Procédé selon la revendication 1, dans lequel ledit angle s'élève à environ 140°.

3. Procédé selon la revendication 1 ou 2, et également pour mesurer la concentration des graisses dans ledit produit laitier, et comprenant en outre la détection de la lumière diffusée par les particules de graisses dans le produit laitier formant un angle dans la plage d'environ 5° à environ 45° par rapport audit faisceau de lumière et la génération d'un deuxième signal de détection qui est représentatif pour la concentration des graisses dans ledit échantillon.

4. Procédé selon la revendication 3, dans lequel l'angle mentionné en dernier lieu s'élève à environ 40°.

5. Procédé selon la revendication 3 ou 4, et comprenant en outre l'étape consistant à générer un signal représentatif du rapport caséine/graisses dans ledit échantillon en réponse au signal de détection mentionné en premier lieu et audit deuxième signal de détection.

6. Procédé selon l'une quelconque des revendications précédentes, et comprenant en outre l'étape consistant à diluer le produit laitier avec de l'eau pour obtenir ledit échantillon.

7. Procédé selon l'une quelconque des revendications précédentes, dans lequel ledit faisceau de lumière (32) possède une longueur d'onde dans la plage d'environ 780 à 1100 nanomètres.

8. Appareil pour mesurer la concentration de caséine dans un produit laitier, **caractérisé par** une source de lumière (30) pour générer un faisceau de lumière polarisée (32) possédant une direction de polarisation ; une cellule de diffusion (20) disposée de façon à recevoir ledit faisceau de lumière (32), pour la réception d'un échantillon dudit produit laitier, ladite cellule de diffusion (20) possédant une première fenêtre (22) et une deuxième fenêtre (24), la deuxième fenêtre (24) étant plus éloignée de ladite source de lumière (30) que la première fenêtre (22), une normale à une surface externe de la deuxième fenêtre (24) formant un angle de Brewster ou approximativement un angle de Brewster par rapport audit faisceau de lumière (32) et la direction de polarisation dudit faisceau de lumière (32) étant parallèle à un plan défini par ledit faisceau de lumière (32) et la normale à la surface externe de la deuxième fenêtre (24) ; et un détecteur de lumière (42) qui est disposé en formant un angle dans la plage d'environ 130° à environ 160° par rapport audit faisceau de lumière (32) et qui sert à détecter la lumière diffusée par la caséine dudit produit laitier et à générer un signal de détection qui est représentatif pour la concentration de ladite caséine dans ledit échantillon.

9. Appareil selon la revendication 8, dans lequel ledit angle s'élève à environ 140°.

10. Appareil selon la revendication 8 ou 9, et comprenant en outre un analyseur de données (220), sensible au signal de détection, pour déterminer la concentration de caséine dans ledit échantillon.

11. Appareil selon la revendication 8 ou 9, et comprenant en outre : un autre détecteur de lumière (40) disposé en formant un autre angle dans la plage d'environ 5° à environ 45° par rapport audit faisceau de lumière (32) pour détecter la lumière diffusée par les particules de graisses dans ledit produit laitier et pour générer un deuxième signal de détection qui est représentatif pour la concentration desdites particules de graisse dans ledit échantillon.

12. Appareil selon la revendication 11, dans lequel ledit autre angle s'élève à environ 40°.

13. Appareil selon la revendication 11 ou 12, et comprenant en outre un analyseur de données (220) sensible au signal de détection mentionné en premier lieu et audit deuxième signal de détection, pour générer un signal représentatif du rapport caséine/graisses dans ledit échantillon.

14. Appareil selon l'une quelconque des revendications 8 à 13, dans lequel ladite source de lumière (30) comprend un laser (110) et un dispositif de polarisation (150).

15. Appareil selon l'une quelconque des revendications 8 à 13, dans lequel ladite source de lumière (110) comprend une diode laser (110) qui sert à générer ledit faisceau de lumière polarisée (32).

16. Appareil selon l'une quelconque des revendications 8 à 15, dans lequel ladite source de lumière (30) sert à émettre ledit faisceau de lumière (32) avec une longueur d'onde dans la plage d'environ 780 à 1100 nanomètres.

17. Appareil selon l'une quelconque des revendications 8 à 16, dans lequel la première fenêtre (22) et la deuxième fenêtre (24) de ladite cellule de diffusion (20) possèdent des surfaces parallèles.

18. Appareil selon l'une quelconque des revendications 8 à 17, dans lequel ladite cellule de diffusion (20) englobe un orifice d'entrée et un orifice de sortie pour permettre audit échantillon de circuler à travers la cellule de diffusion (20).

19. Appareil selon l'une quelconque des revendications 8 à 18, dans lequel la surface interne de la deuxième fenêtre (24) est parallèle à la surface externe de la deuxième fenêtre (24).

20. Appareil selon l'une quelconque des revendications 8 à 18, dans lequel la perpendiculaire à la surface interne (186') de la deuxième fenêtre (124') pour la mise en contact avec ledit échantillon forme un angle de Brewster ou approximativement un angle de Brewster par rapport audit faisceau de lumière (112), et dans lequel la deuxième fenêtre (124') est de configuration cunéiforme.

21. Appareil selon l'une quelconque des revendications 8 à 20, et comprenant en outre un moyen pour diluer ledit produit laitier avec de l'eau pour obtenir ledit échantillon.
